# EUROPÄISCHE PATENTANMELDUNG

(11) **EP 1 695 728 A1**
(43) Veröffentlichungstag der Anmeldung: **30.08.2006**
(21) Anmeldenummer: 05003882.7
(22) Anmeldetag: 23.02.2005
(51) Int. Cl.: A61M 16/00

(54) **Bauteil für eine Inhalationsvorrichtung, Inhalationsvorrichtung mit diesem Bauteil und Steuerverfahren für ein solches Bauteil**

(71) Anmelder: Activaero GmbH, 35285 Gemünden (DE)
(72) Erfinder: Müllinger, Bernhard, 80634 München (DE); Fischer, Axel, 34630 Moischeid (DE); Körber, Dorothee, 86157 Augsburg (DE); Wenker, Andreas, 86899 Landsberg am Lech (DE); Kolb, Tobias, 80687 München (DE); Roeder, Sascha, 80992 München (DE); Scheuch, Gerhard, Dr., 35288 Wohratal (DE)
(74) Vertreter: Vossius & Partner

(57) **Zusammenfassung**

Die Erfindung betrifft ein Bauteil (10) für eine Inhalationsvorrichtung (1) mit einer ersten Lufteinlassöffnung (11) und einer ersten Luftauslassöffnung (12), die über einen ersten Strömungskanal (13) für die Inhalation verbunden sind, einer zweiten Lufeinlassöffnung (14) und einer zweiten Luftauslassöffnung (15) die über einen zweiten Strömungskanal (16) für die Exhalation verbunden sind und einem in dem zweiten Strömungskanal angeordneten Filter (17), wobei die Exhalation nur über den zweiten Strömungskanal erfolgen kann.

## Beschreibung

Die vorliegende Erfindung betrifft ein Bauteil für eine Inhalationsvorrichtung, insbesondere für eine Inhalationsvorrichtung zur Inhalation von toxischen Wirkstoffen. Ferner betrifft die vorliegende Erfindung eine Inhalationsvorrichtung mit einem erfindungsgemäßen Bauteil sowie ein Steuerverfahren für das erfindungsgemäße Bauteil.

Die Verwendung des Inhalationswegs für die Applikation von Medikamenten gewinnt zunehmend an Bedeutung. Dabei werden außer dem Einsatz neuer lokal wirkender Medikamente für die Therapie von Lungenerkrankungen auch neue Therapiestrategien entwickelt, die die Lunge als Eingangsorgan für systemisch wirkende Substanzen verwenden.

Unter Umständen sind durch Inhalation toxische Wirkstoffe zu applizieren. Beispielsweise können bei einem Patienten mit Lungenkarzinom Zytostatika (z.B. Cisplatin) oder Zytokine verabreicht werden. Bei der Verabreichung von toxischen Wirkstoffen besteht immer die Notwendigkeit, die Belastung der Umwelt oder evtl. anwesender weiterer Personen mit diesen toxischen Wirkstoffen auszuschließen bzw. so gering wie möglich zu halten. Beispielsweise können während des Ausatemvorgangs Reste des toxischen Wirkstoffs wieder aus der Lunge des Patienten ausgeatmet werden. Herkömmliche Inhalationsgeräte tragen diesem Umstand nicht genügend Rechnung oder sind sehr unkomfortabel.

Der Erfindung liegt die Aufgabe zugrunde, die Inhalation toxischer Wirkstoffe unter Ausschaltung oder Verringerung möglicher Risiken oder einer Kontamination der Umgebung des behandelten Patienten zu ermöglichen. Diese Aufgabe wird mit einem Bauteil für eine Inhalationsvorrichtung, eine Inhalationsvorrichtung und einem Steuerverfahren gemäß der Patentansprüche gelöst.

Gemäß einem ersten Aspekt betrifft die vorliegende Erfindung ein Bauteil für eine Inhalationsvorrichtung. Das erfindungsgemäße Bauteil weist einen ersten Strömungskanal für die Inhalation auf, der sich zwischen einer ersten Lufteinlassöffnung und einer ersten Luftauslassöffnung erstreckt. Ferner ist ein zweiter Strömungskanal für die Exhalation vorgesehen, der sich zwischen einer zweiten Lufteinlassöffnung und einer zweiten Luftauslassöffnung erstreckt. Vorzugsweise fallen die erste Luftauslassöffnung und die zweite Lufteinlassöffnung zusammen, so dass ein und dieselbe Öffnung als Luftauslassöffnung für den ersten Strömungskanal und als Lufteinlassöffnung für den zweiten Strömungskanal dient. Alternativ dazu fallen die erste Lufteinlassöffnung und die erste Luftauslassöffnung, sowie die zweite Lufteinlassöffnung und die zweite Luftauslassöffnung zusammen, so dass lediglich eine erste und eine zweite Öffnung vorhanden sind, die die beiden Strömungskanäle darstellen.

Das erfindungsgemäße Bauteil weist ferner ein Filter auf, das in dem zweiten Strömungskanal angeordnet ist, bzw. dem zweiten Strömungskanal/der zweiten Öffnung zugeordnet ist, und beispielsweise in Verlängerung des zweiten Strömungskanals, d.h. stromabwärts, vorgesehen ist. Erfindungsgemäß kann die Exhalation vorzugsweise nur über den zweiten Strömungskanal erfolgen. Vorzugsweise wird der zweite Strömungskanal dann freigegeben und der erste Strömungskanal verschlossen, wenn die Inhalation durch den Patienten beendet wird, sei es durch Abbruch oder durch Zeitablauf am Ende der notwendigen Inhalationsdauer oder am Ende des Atemzuges.

Wenn das erfindungsgemäße Bauteil in einer Inhalationsvorrichtung verwendet wird, kann der Patient über den ersten Strömungskanal inhalieren. Dazu ist die erste Lufteinlassöffnung stromaufwärts mit einem Vernebler verbindbar, der den zu applizierenden Wirkstoff beispielsweise in Aerosolform in einen Druckluftstrom einbringt. Die Inhalation durch den Patienten erfolgt dann über ein Mundstück, das an der ersten Luftauslassöffnung abstromseitig des ersten Strömungskanals angeschlossen ist. Die Exhalation erfolgt in entgegengesetzter Richtung über das Mundstück, jedoch dann nicht über den ersten Strömungskanal zurück in Richtung Vernebler, sondern über den zweiten Strömungskanal zu dem Filter, das in der Ausatemluft vorhandene Restbestände des toxischen Wirkstoffs herausfiltert.

Vorzugsweise ist an das Filter stromabwärts eine Absaugvorrichtung angeschlossen. Dies hat den Vorteil, dass bei Beendigung der Inhalation nicht nur die ausgeatmete Luft des Patienten gefiltert wird, sondern auch aktiv der zweite Strömungskanal und das Mundstück abgesaugt und damit entleert werden, so dass auch nach Absetzen des Mundstücks keine toxischen Wirkstoffe aus der Mundstücköffnung in die Umgebung gelangen können. Vorzugsweise ist in diesem Absaugkanal ein Exhalationspuffer vorhanden, der im Falle zu niedriger Absaugraten die ausgeatmete Luft zwischenspeichert. Der Exhalationspuffer wird dann beispielsweise während des nächsten Inhalationsschrittes weiter entleert.

Vorzugsweise bilden der erste Strömungskanal und der zweite Strömungskanal ein 3-Wege-Ventil. Die drei Öffnungen entsprechen (i) dem ersten Lufteinlass, (ii) dem ersten Luftauslass/zweiten Lufteinlass und (iii) dem zweiten Luftauslass. Alternativ dazu sind zwei getrennte Strömungskanäle vorgesehen, wobei beispielsweise an der ersten Luftauslassöffnung ein Einwegventil vorgesehen ist, das lediglich eine Inhalation, also einen Luftstrom zum Patienten, zulässt, und an der zweiten Lufteinlassöffnung ebenfalls ein Einwegventil vorgesehen ist, das lediglich eine Exhalation zulässt. Dadurch ist sichergestellt, dass die Exhalation nur über den zweiten Strömungskanal erfolgt.

Für den Fall, dass die erste Lufteinlassöffnung und die erste Luftauslassöffnung, sowie die zweite Lufteinlassöffnung und die zweite Luftauslassöffnung zusammen fallen, so dass lediglich eine erste und eine zweite Öffnung vorhanden sind, stellt das erfindungsgemäße Bauteil vorzugsweise eine Inhalationsmaske dar.

In einer bevorzugten Ausführungsform ist ferner abstromseitig von der ersten Luftauslassöffnung bzw. der zweiten Lufteinlassöffnung ein Schutzelement vorgesehen, das vorzugsweise selbst-expandierend ist, beispielsweise ein Faltenbalg. Dieses Schutzelement umgibt die erste Luftauslassöffnung bzw. die zweite Lufteinlassöffnung oder ein gegebenenfalls vorhandenes Mundstück. Dabei ist das Schutzelement dichtend beispielsweise mit dem Ende des Mundstücks verbunden, dass der Mundstückspitze gegenüber liegt. Das Schutzelement ist im Ruhezustand expandiert, ist aber bei Verwendung der Inhalationsvorrichtung komprimierbar. Im expandierten Zustand erstreckt sich das Schutzelement über die Spitze des Mundstücks hinaus, wie ein Auffangbehältnis. Wenn somit der Benutzer die Inhalationsvorrichtung vom Mund entfernt, expandiert das Schutzelement automatisch, um dadurch seine Schutzfunktion zu entfalten: die kontaminierte Luft bleibt im vorstehenden Schutzelement gefangen und kann nicht an die Umgebung entweichen, sondern wird abgesaugt und gefiltert. Um dem Benutzer das Ausatmen zu ermöglichen bzw. zu erleichtern, bevor der Inhalationsvorgang beginnt, oder in einer Pause, weist das Schutzelement vorzugsweise ein Ausatemventil auf. Somit kann der Benutzer in das Innere des Schutzelementes ausatmen und die Luft entweicht dann über das Ausatemventil. Besonders bevorzugt erstreckt sich das Schutzelement über das gesamte erfindungsgemäße Bauteil (vergleichbar mit einem Ballon), so dass alle eventuellen Undichtigkeiten abgedeckt werden und keine kontaminierte Luft in die Umgebung entweichen kann, sondern vom Schutzelement abgehalten wird.

Der Überstand des Schutzelementes beträgt vorzugsweise zwischen 5 und 100 mm. Der Absaugfluss beträgt hingegen vorzugsweise zwischen 15 und 80 l/min. Je größer der Überstand, desto geringer kann der Absaugfluss sein, bei dem noch kein Austritt des Aerosols erfolgt.

Gemäß einer alternativen Ausführungsform weist das Bauteil eine Inhalationsmaske auf, die derart gestaltet und dimensioniert ist, dass sie den Mund und die Nase des Benutzers überdecken kann, so dass jegliche über den Mund oder die Nase ausgeatmete Luft in das Maskeninnere gelangt. Beispielsweise ist diese eine aus der Beatmung bekannte CPAP (Continuous Positive Airway Pressure) Maske. Gemäß einer ersten Alternative weist die Inhalationsmaske eine Öffnung auf, an der beispielsweise eine Schlauchverbindung zwischen den Strömungskanälen und der Inhalationsmaske endet (die Strömungskanäle können auch direkt in das Maskeninnere münden). Die Inhalationsmaske schließt hier vorzugsweise bündig mit der Schlauchverbindung ab, so dass die Inhalationsmaske wie ein trichterförmiges Element die Schlauchverbindung verlängert. In dieser Alternative ist ein Mundstück, das der Patient in den Mund nimmt, nicht zwingend vorgesehen. Alternativ dazu ist ein Mundstück vorhanden und durchdringt die Inhalationsmaske und ragt in den Maskeninnenraum, der zwischen der Inhalationsmaske und dem Gesicht des Benutzers gebildet wird, hinein. Vorzugsweise ragt das Mundstück derart weit in die Inhalationsmaske hinein, dass der Benutzer das Mundstückende bzw. die Mundstückspitze zur Inhalation/Exhalation leicht in den Mund nehmen kann. In dieser Ausführungsform sieht eine bevorzugte Ausgestaltung vor, dass das Mundstück relativ zur Inhalationsmaske verschiebbar ist. Dabei ist das Mundstück zwischen einer ersten Position (der Inhalationsstellung), in der der Benutzer das Mundstück in den Mund nehmen kann, und einer zweiten Position (der Exhalationsstellung), in der das Mundstück in den Maskeninnenraum zurückgezogen ist, verschiebbar. Dies hat den Vorteil, dass der Benutzer das Mundstück zur Inhalation in den Mund nehmen kann, zur Exhalation dieses aber freigibt und zurückzieht, so dass die Exhalation nicht in Richtung des Verneblers erfolgt, sondern in den Maskeninnenraum. Außerdem kann der Benutzer so zwischendurch einatmen ohne zu inhalieren.

Vorzugsweise ist hierzu ein flexibles Element als Bindeglied zwischen dem Mundstück und der Maske vorgesehen. Mehr bevorzugt ist das flexible Element selbst-expandierend, so dass das Mundstück, wenn es vom Patienten freigegeben wurde, automatisch durch die Expansion des flexiblen Elements zurückgezogen wird.

Vorzugsweise weist die Inhalationsmaske eine Absaugöffnung auf. Diese Absaugöffnung ist vorzugsweise mit einem Filter und einer damit verbundenen Absaugeinrichtung verbunden. Damit kann in den Maskeninnenraum ausgeatmete Luft über das Filter abgesaugt und gereinigt werden.

Gemäß einer weiter bevorzugten Ausführungsform ist die Inhalationsmaske im Umfangsbereich derart ausgebildet, dass im Falle eines Unterdrucks im Maskeninnenraum ein Druckausgleich über den Umfangsbereich der Inhalationsmaske möglich ist. Dies bedeutet, dass der Umfangsbereich der Inhalationsmaske so ausgestaltet ist, dass er nicht absolut dichtend an der Haut des Patienten anliegt, sondern den Zufluss von Luft in den Maskeninnenraum im Falle eines Unterdrucks zulässt. Dies ist besonders vorteilhaft, wenn eine Absaugvorrichtung die im Inhalationsmaskeninnenraum vorhandene Luft über eine entsprechende Öffnung absaugt, denn dann kann die Absaugvorrichtung, beispielsweise als Sicherheitsvorkehrung, kontinuierlich betrieben werden, und im Falle eines Unterdrucks strömt Umgebungsluft zum Druckausgleich in den Maskeninnenraum. Mit dem toxischen Wirkstoff kontaminierte Ausatemluft kann jedoch nicht über den Umfangsbereich der Inhalationsmaske entweichen, da die Absaugvorrichtung für deren Absaugung sorgt.

Alternativ zu der Möglichkeit, Luftzufluss über den Maskenrand zuzulassen, weist die Maske Einwegventile auf, die für den nötigen Druckausgleich sorgen. Diese sind vorzugsweise im vorderen, also vom Mund des Benutzers entfernten Bereich der Maske vorgesehen.

Die Inhalationsmaske ist vorzugsweise ballonförmig ausgebildet. Vorzugsweise durchdringt das Mundstück die derart ballonförmig ausgebildete Inhalationsmaske in einem ersten Bereich, der dem Gesicht des Benutzers abgewandt ist, und erstreckt sich in den Maskeninnenraum. Ein diesem Bereich gegenüberliegender zweiter Bereich, der bei der Inhalation dem Gesicht des Benutzers zugewandt ist, ist vor der Inhalation verschlossen und wird erst zu Beginn der Inhalation dadurch von der Mundstückspitze des Mundstücks durchdrungen, dass der Benutzer diesen Maskenbereich an sein Gesicht anlegt und das im Inneren der Inhalationsmaske befindliche Mundstückende zum Mund verschiebt.

Das erfindungsgemäße Bauteil ist vorzugsweise Bestandteil des Aerosolgenerators der Inhalationsvorrichtung.

Ein zweiter erfindungsgemäßer Aspekt betrifft eine Inhalationsvorrichtung mit einem ersten Strömungskanal für die Inhalation und einem zweiten Strömungskanal für die Exhalation, wobei Mittel vorgesehen sind, die verhindern, dass vor, während und nach der Inhalation durch den Benutzer von der Inhalationsvorrichtung abgegebener Wirkstoff in die Umgebung entweicht.

Gemäß einer bevorzugten Ausführungsform des zweiten Aspekts der vorliegenden Erfindung wird eine Inhalationsvorrichtung mit einem erfindungsgemäßen Bauteil gemäß dem ersten Aspekt sowie mit einem Vernebler, der mit der ersten Lufteinlassöffnung verbunden ist und einem Mundstück, das mit der ersten Luftauslassöffnung und der zweiten Lufteinlassöffnung verbunden ist, bereitgestellt. Vorzugsweise weist die Inhalationsvorrichtung eine Absaugeinrichtung auf, die mit der zweiten Luftauslassöffnung des Bauteils derart verbunden ist, dass ausgeatmete Luft über das Filter des Bauteils abgesaugt wird.

Alternativ dazu weisen die Verhinderungsmittel eine Inhalationsmaske mit einer ersten Öffnung für die Inhalation und einer Absaugöffnung auf, ferner mit einem mit der ersten Öffnung verbundenen Vernebler und einer mit der Absaugöffnung verbundenen Absaugeinrichtung.

Gemäß einem dritten Aspekt der Erfindung wird ein Steuerverfahren für ein erfindungsgemäßes Bauteil bereitgestellt, wobei bei Erkennung einer Beendigung des Inhalationsvorgangs der zweite Strömungskanal für die Exhalation freigegeben wird. Vorzugsweise wird dieser Strömungskanal über einen Drucksensor an der Maske gesteuert.

Vorzugsweise schaltet die erfindungsgemäße Inhalationsvorrichtung die Verneblung des Wirkstoffs nur dann ein, wenn der Patient oder der Benutzer am Mundstück einen leichten Unterdruck erzeugt und so der Wille des Benutzers zur Inhalation erkennbar ist. Ebenso schaltet die Verneblung vorzugsweise unverzüglich ab, wenn der Unterdruck am Mundstück nachlässt.

Die Absaugung der ausgeatmeten Luft des Benutzers wird vorzugsweise erst dann zugeschaltet, wenn überhaupt vom Vernebler Aerosol erzeugt wird, da lediglich dann die Gefahr besteht, toxische Wirkstoffe an die Umgebung auszuatmen. Dies bedeutet, dass die Absaugung auch erst dann beginnt, wenn der Benutzer/Patient mit der Inhalation beginnt, da dies die Verneblung startet. Sofern der Patient während der Inhalation stoppt, wird die Absaugung fortgeführt, und die Absaugung vorzugsweise sogar erhöht, um umgehend die Strömungskanäle zu entleeren. Bei einer regulären Beendung der Inhalation stoppt zwar die Verneblung des Wirkstoffs, die Absaugung wird jedoch fortgeführt, da nun erst die Exhalation beginnt. Auch nach erfolgter Exhalation durch den Benutzer wird die Absaugung noch für einen gewissen Zeitbereich durchgeführt, um eventuelle Restbestände kontaminierter Ausatemluft abzusaugen.

Die Erfindung wird nun unter Bezugnahme auf die beigefügten Zeichnungen näher erläutert. Es zeigen:
- Fig. 1: den prinzipiellen Aufbau einer Inhalationsvorrichtung für die Inhalation toxischer Medikamente;
- Fig. 2a: ein Bauteil gemäß einer ersten bevorzugten erfindungsgemäßen Ausführungsform;
- Fig. 2b: die Verwendung des Bauteils von Fig. 2a;
- Fig. 3: ein Bauteil gemäß einer zweiten bevorzugten erfindungsgemäßen Ausführungsform;
- Fig. 4: ein Bauteil gemäß einer dritten bevorzugten erfindungsgemäßen Ausführungsform;
- Fig. 5: ein Bauteil einer vierten bevorzugten erfindungsgemäßen Ausführungsform;
- Fig. 6: die Verwendung einer bevorzugten Inhalationsmaske während des Inhalationsvorgangs;
- Fig. 7: die Verwendung einer bevorzugten Inhalationsmaske während der Exhalation;
- Fig. 8: eine schematische Darstellung einer bevorzugten Inhalationsmaske;
- Fig. 9: eine weiter alternative Ausführungsform der Inhalationsmaske;
- Fig. 10: eine weiter alternative Ausführungsform der Inhalationsmaske;
- Fig. 11a: ein Bauteil gemäß einer weiter bevorzugten Ausführungsform während der Inhalation; und
- Fig. 11b: das Bauteil von Fig. 11a während der Exhalation.

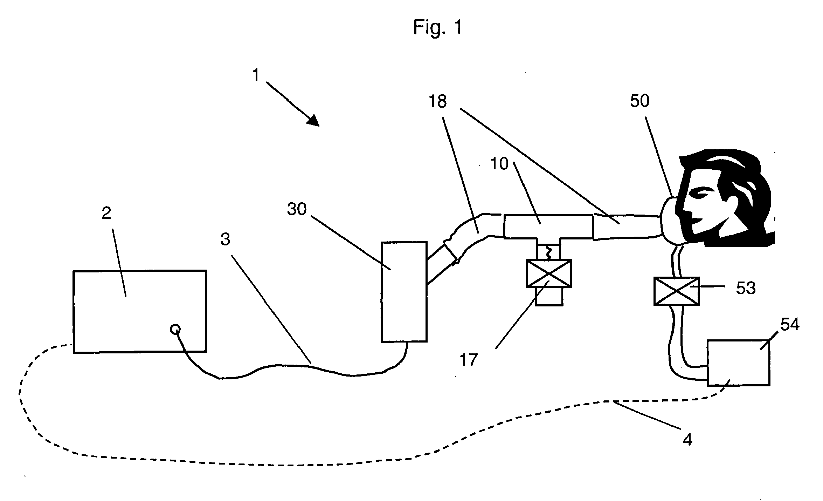

Fig. 1 zeigt den prinzipiellen Aufbau einer erfindungsgemäßen Inhalationsvorrichtung 1 für die Inhalation toxischer Medikamente. Diese Inhalationsvorrichtung weist ein Inhalationsgerät 2 auf, das die Steuerelektronik, Eingabetasten, Display etc. enthält und über ein entsprechendes Verbindungskabel 3 für Luft, Daten und Strom mit einem Vernebler 30 verbunden ist. In dem Vernebler 30 wird der zu applizierende Wirkstoff zerstäubt und in einen Druckluftstrom eingebracht, der über einen entsprechenden Schlauch 18, das erfindungsgemäße Bauteil 10 und einen weiteren Schlauch 18 (oder ein Mundstück (nicht gezeigt)) mit einer Maske 50 verbunden ist. Diese Maske 50 trägt der Patient, Mund und Nase überdeckend, an seinem Gesicht. Das erfindungsgemäße Bauteil 10 weist ein Filter 17 auf, das bei der Inhalation toxischer Wirkstoffe die in der Ausatemluft vorhandenen Restbestände dieser Wirkstoffe aus der Ausatemluft herausfiltert und somit die Ausatemluft reinigt. Entsprechend ist gemäß Fig. 1 vorzugsweise ein weiteres Filter 53 vorgesehen, das über einen entsprechenden Schlauch direkt mit dem Innenraum der Inhalationsmaske 50 verbunden ist. Eine Absaugvorrichtung 54, die ebenfalls von dem Inhalationsgerät 2 über eine Verbindungsleitung 4 angesteuert wird, saugt die ausgeatmete Luft über das Filter 53 aus der Inhalationsmaske 50 heraus.

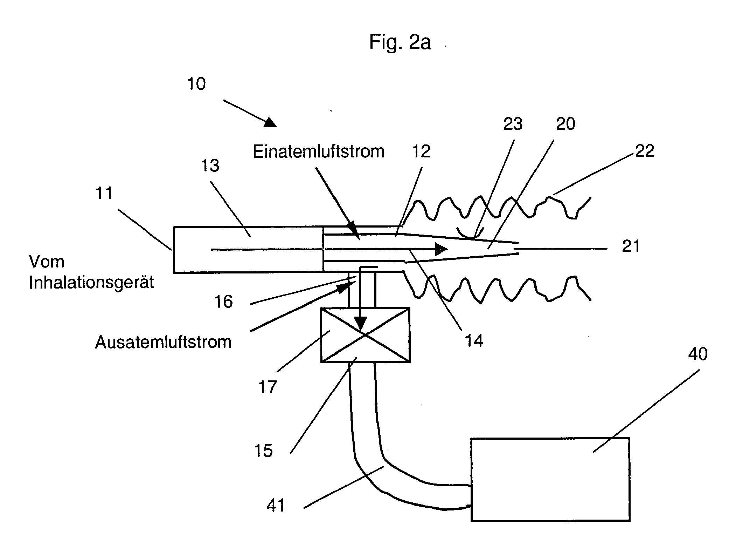

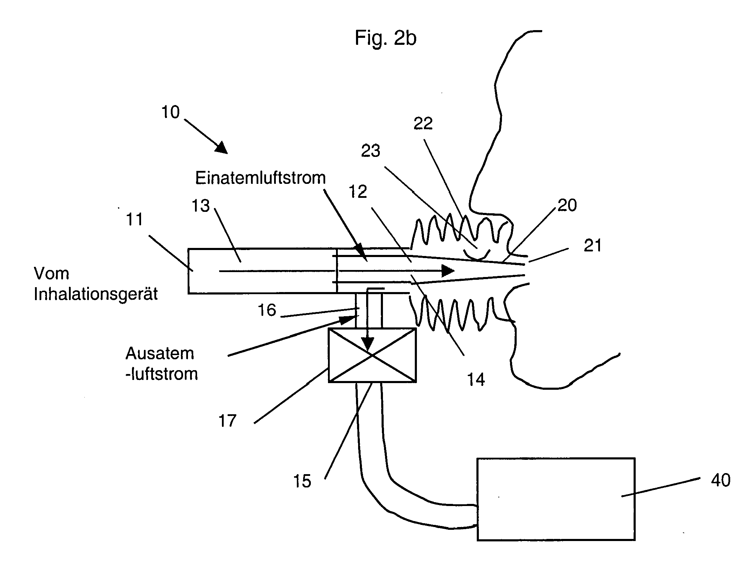

Gemäß Fig. 2a weist das erfindungsgemäße Bauteil 10 einen ersten Strömungskanal 13 zwischen einer ersten Lufteinlassöffnung 11 und einer ersten Luftauslassöffnung 12 auf. Über diesen ersten Strömungskanal 13 erfolgt die Inhalation vom Vernebler und über das Mundstück 20. Der Patient nimmt dazu das Mundstückende bzw. die Mundstückspitze 21 in den Mund, wie in Fig. 2b gezeigt. Für die Exhalation ist ein zweiter Strömungskanal 16 vorgesehen, der sich zwischen einer zweiten Lufteinlassöffnung 14 und einer zweiten Luftauslassöffnung 15 erstreckt. In der in den Fig. 2a und 2b gezeigten bevorzugten Ausführungsform fallen die erste Luftauslassöffnung 12 und die zweite Lufteinlassöffnung 14 zusammen. In dieser Ausführungsform ist das erfindungsgemäße Bauteil 10 als 3-Wege-Ventil ausgebildet, das eine Inhalation über den ersten Strömungskanal 13, eine Exhalation jedoch lediglich über den zweiten Strömungskanal 16 zulässt. Im zweiten Strömungskanal 16 oder diesem zugeordnet ist ein Filter 17 vorgesehen, das die ausgeatmete Luft reinigt und dekontaminiert. Gemäß der bevorzugten Ausführungsform von Fig. 2a/2b ist an den zweiten Strömungskanal 16 über einen Schlauch 41 eine Absaugvorrichtung 40 angeschlossen, die die ausgeatmete Luft absaugt.

In der bevorzugten Ausführungsform von Fig. 2a ist ferner ein Schutzelement 22 vorgesehen, das vorzugsweise selbst-expandierend ist, beispielsweise ein Faltenbalg. Dieses Schutzelement 22 ist im Ruhezustand expandiert, wie in Fig. 2a gezeigt, ist aber bei Verwendung der Inhalationsvorrichtung komprimierbar, wie in Fig. 2b gezeigt. Im expandierten Zustand erstreckt sich das Schutzelement 22 über die Spitze 21 des Mundstücks 20 hinaus. Wenn somit der Benutzer die Inhalationsvorrichtung vom Mund entfernt, expandiert das Schutzelement 22 automatisch, um dadurch seine Schutzfunktion zu entfalten: kontaminierte Luft bleibt im Schutzelement 22 gefangen und kann nicht an die Umgebung entweichen, sondern wird abgesaugt und gefiltert. Um dem Benutzer das Ausatmen zu ermöglichen bzw. zu erleichtern, bevor der Inhalationsvorgang beginnt, oder in einer Pause, weist das Schutzelement 22 vorzugsweise ein Ausatemventil 23 auf.

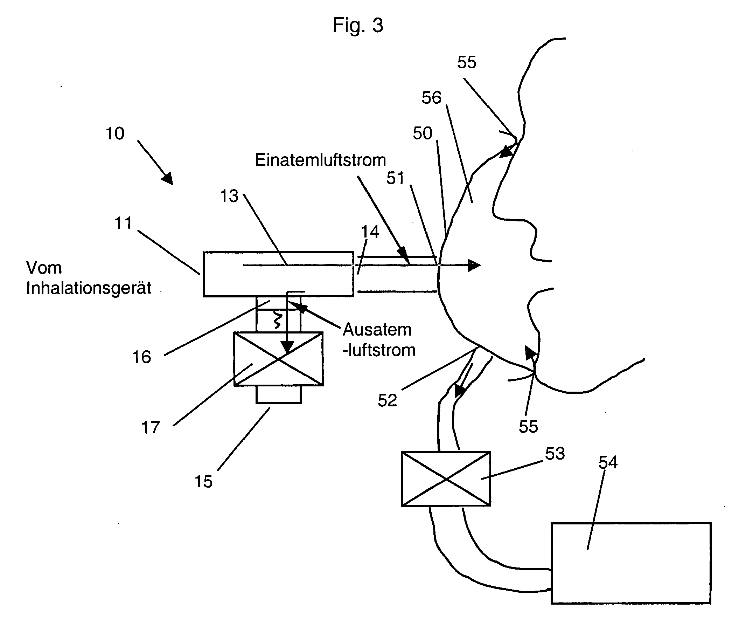

Eine alternative bevorzugte Ausführungsform des erfindungsgemäßen Bauteils ist in Fig. 3 gezeigt. Hier ist im Unterschied zu dem Bauteil von Fig. 2 kein Mundstück 20 vorgesehen, das der Patient in den Mund nimmt. Vielmehr endet der Inhalationsluftstrom in einer Inhalationsmaske 50, die derart dimensioniert ist, dass sie Mund und Nase des Patienten überdeckt. Ein "Mundstück" bzw. eine Schlauchverbindung erstreckt sich von der ersten Luftauslassöffnung 12 bis zu einer Öffnung 51 in der Inhalationsmaske 50. Ferner ist in der Inhalationsmaske 50 eine zweite Öffnung 52 vorgesehen, die den Inhalationsmaskeninnenraum 56 über ein Filter 53 mit einer Absaugvorrichtung 54 verbindet. Über diesen Kanal kann ausgeatmete Luft abgesaugt werden. Überdies wird ausgeatmete Luft über das erfindungsgemäße Bauteil 10 und insbesondere über den zweiten Strömungskanal 16 und das Filter 17 abgeführt.

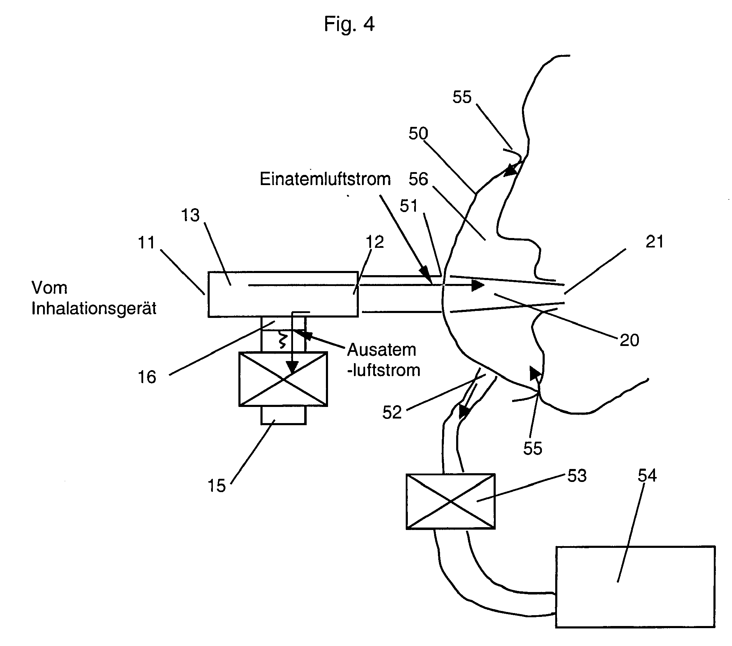

Fig. 4 zeigt eine zu Fig. 3 ähnliche Ausführungsform. In der Ausführungsform von Fig. 4 ist jedoch noch zusätzlich ein Mundstück 20 vorgesehen. Das Mundstück 20 erleichtert dem Patienten die Inhalation, da er dieses an dessen Ende 21 in den Mund nehmen kann und bequem lediglich über diesen Strömungskanal inhaliert. Die Ausatmung erfolgt in dieser Ausführungsform beispielsweise über die Nase, so dass der Patient das Mundstück im Mund belassen kann. Derart ausgeatmete Luft wird wiederum über das Filter 53 von der Absaugvorrichtung 54 direkt aus dem Maskeninneren 56 abgesaugt. Um jedoch im Falle eines Unterdrucks im Maskeninneren 56 zu verhindern, dass die Maske 50 auf das Gesicht des Patienten gezogen wird, ist die Inhalationsmaske 50 im Umfangsbereich 55 derart ausgebildet, dass dort ein Lufteinlass möglich ist. Wie oben erläutert sind alternativ dazu Einwegventile vorgesehen, die für einen notwendigen Druckausgleich sorgen. Sobald also beispielsweise über die Absaugvorrichtung 54 ein Unterdruck im Maskeninneren 56 entsteht, kann Umgebungsluft über den Umfangs- bzw. Randbereich 55 oder ein Einwegventil (nicht gezeigt) nachfließen. Entsprechendes gilt für die in Fig. 3 gezeigte bevorzugte Ausführungsform.

Wenn in der Ausführungsform von Fig. 4 nicht nur über die Nase sondern auch oder ausschließlich über den Mund exhaliert wird, wird die exhalierte Luft über den zweiten Strömungskanal 16 und das Filter 17 abgeführt und gereinigt.

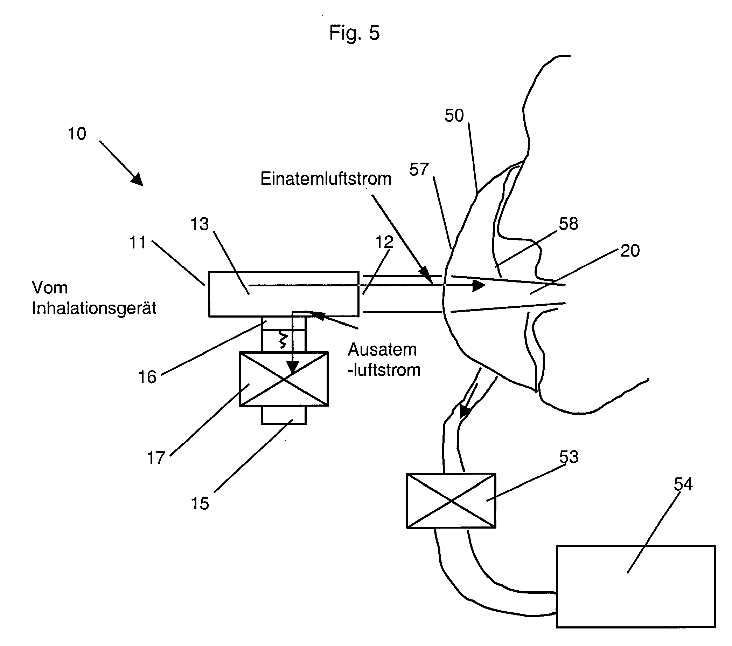

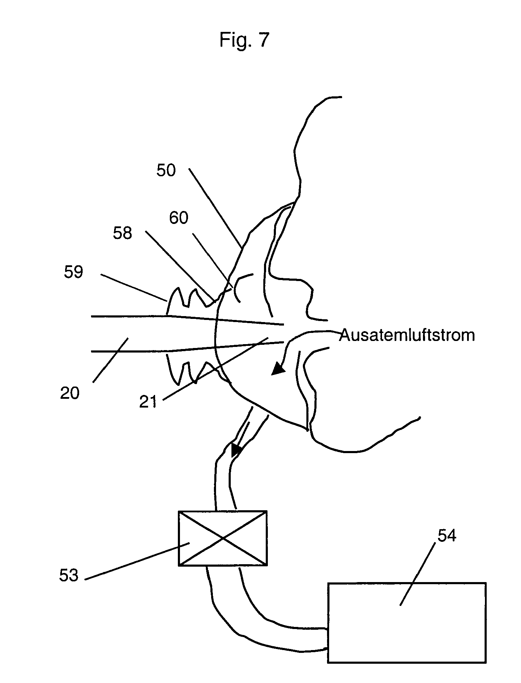

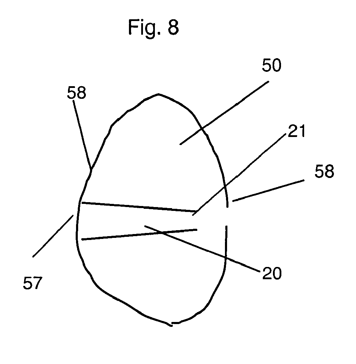

Fig. 5 zeigt eine weitere bevorzugte Ausführungsform des erfindungsgemäßen Bauteils 10. Gemäß dieser bevorzugten Ausführungsform wird eine besonders bevorzugte Inhalationsmaske 50 verwendet, die in Fig. 8 näher dargestellt ist. Diese Inhalationsmaske 50 ist ballonförmig ausgebildet und wird in einem ersten Bereich 57 vom Mundstück 20 durchdrungen. Dieser erste Bereich 57 ist der Bereich der Inhalationsmaske 50, der während der Inhalation vom Gesicht des Patienten abgewandt ist. Der gegenüberliegende Bereich 58 ist hingegen während der Inhalation dem Gesicht des Patienten zugewandt. Vor der Inhalation ragt der Bereich 58 wie in Fig. 7 gezeigt über die Mundstückspitze 21 hinaus. Auch dies stellt eine Schutzfunktion wie oben im Zusammenhang mit Fig. 2 erläutert bereit. Eventuell ausströmende kontaminierte Luft wird in der Maske 50 gefangen und abgesaugt. Sobald jedoch der Patient die Inhalationsmaske anlegt und an das Gesicht drückt, überragt die Spitze bzw. das Ende 21 des Mundstücks 20 den Bereich 58 der Inhalationsmaske, so dass der Patient das Mundstück in den Mund nehmen kann. Der Bereich 58 wird dabei lediglich auf das Mundstück 20 geschoben. Dies ist in Fig. 5 gezeigt.

Fig. 5 zeigt die erfindungsgemäße Inhalationsmaske bereits im aufgesetzten Zustand. Ansonsten ist diese bevorzugte Ausführungsform identisch zu der von Fig. 4, so dass auf die Erläuterungen zu Fig. 4 verwiesen wird.

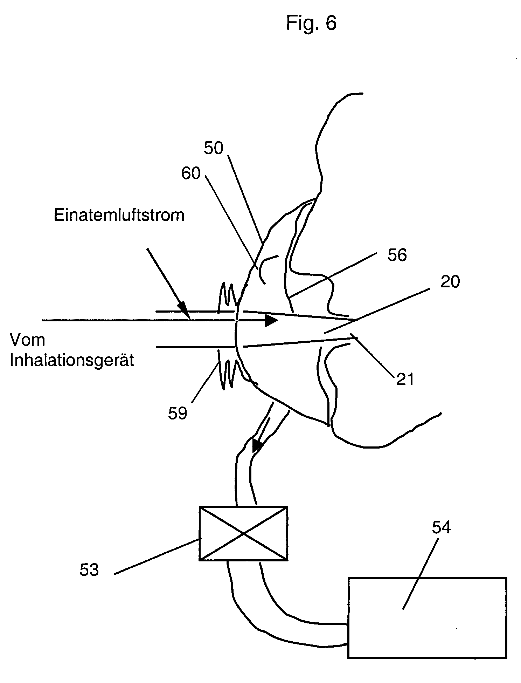

Die Figuren 6 und 7 zeigen eine weitere Variante der Ausbildung von Inhalationsmaske 50 und Mundstück 20. Gemäß dieser bevorzugten Ausführungsform ist das Mundstück 20 relativ zur Inhalationsmaske 50 flexibel gelagert bzw. verschiebbar angeordnet. Fig. 6 zeigt das Mundstück relativ zur Inhalationsmaske in einer Position, in der der Patient inhaliert. Zur Erleichterung der Ausatmung ist jedoch das Mundstück zurückziehbar, d.h. vom Patienten entfernbar, wie in Fig. 7 gezeigt. Der Patient lässt dazu das Mundstückende 21 los und es wird ausreichend weit in den Maskeninnenraum 56 zurückgezogen, so dass der Patient bequem ausatmen kann. Die Ausatemluft wird dann beispielsweise über die Absaugvorrichtung 54 abgesaugt. Zur flexiblen bzw. verschiebbaren Lagerung des Mundstücks 20 ist wie in den Figuren 6 und 7 gezeigt beispielsweise ein Faltenbalg 59 oder ähnliches flexibles Element vorgesehen. Wenn der Patient wie in Fig. 6 gezeigt das Mundstück 20 in den Mund schiebt wird dieses flexible Element 59 zusammengedrückt. Im Falle eines selbst-expandierbaren flexiblen Elementes 59 zieht sich das Mundstück 20 automatisch zurück, wenn es der Patient freigibt.

Die Ausführungsformen von Fig. 6 und 7 zeigen auch das zum Druckausgleich vorhandene Einwegeventil 60 der Maske 50.

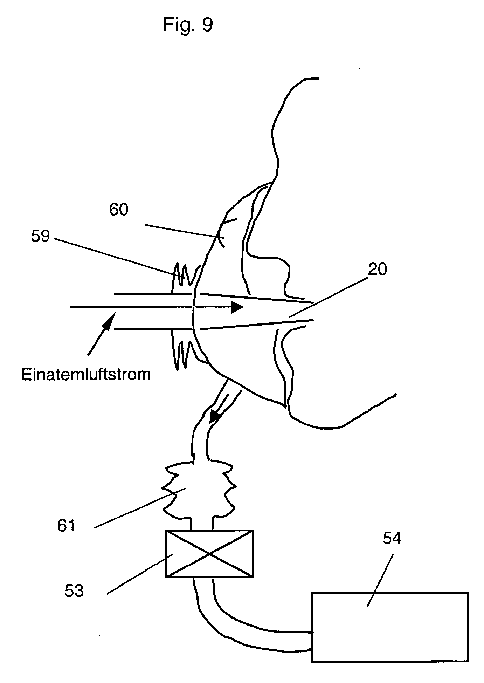

Eine weitere Alternative ist in Fig. 9 gezeigt. Diese entspricht im wesentlichen der Ausführungsform von Fig. 6. In Fig. 9 ist jedoch zusätzlich ein Pufferelement 61 gezeigt, dass als Ausatempuffer bzw. Exhalationsbeutel dient. Im Falle einer zu geringen Absaugrate ist so sichergestellt, das der Patient trotzdem normal ausatmen kann. Nicht abgesaugte exhalierte Luft wird dann im Ausatempuffer gepuffert und verzögert abgesaugt, während der Patient wieder inhaliert.

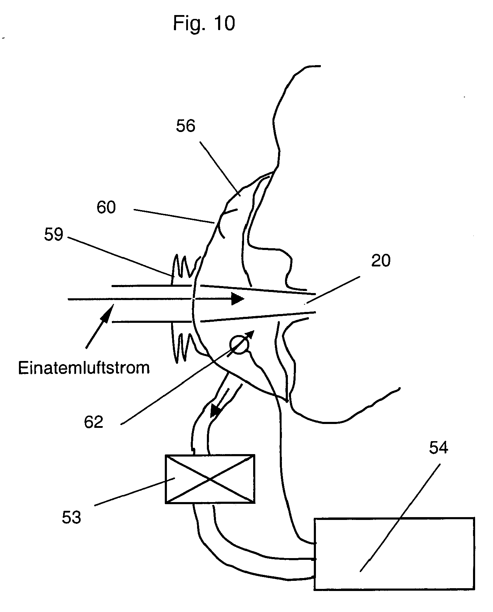

In der Ausführungsform von Fig. 10 ist weiterhin ein Drucksensor 62 vorgesehen. Dieser Drucksensor misst den Druck im Maskeninneren 56 und liefert das Ergebnis an die Absaugvorrichtung 54. Diese ist dann in der Lage, eine druckabhängige bzw. druckgeregelte Absaugrate einzustellen.

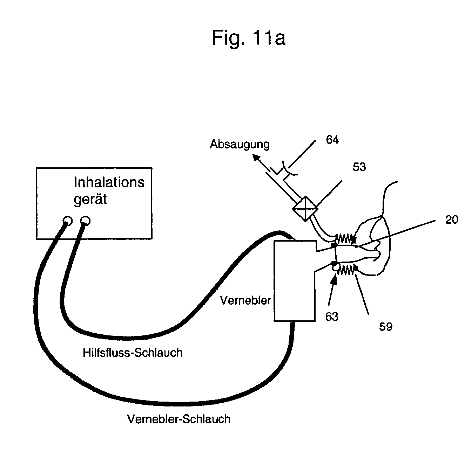

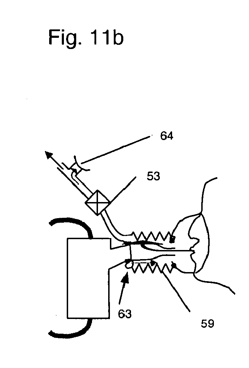

Fig. 11a und 11b zeigen eine alternative Anordnung der Absaugung am Beispiel der Ausführungsform der Fig. 6 und 7. Hier setzt die Absaugeinrichtung an der Verbindungsstelle von Mundstück 20 und flexiblen Element 59 an. Auch ein Inspirationsventil 63 befindet sich in diesem Bereich. Diese erlaubt einen Druckausgleich während der Exhalation sowie eine Einatmung vor Beginn des Inhalationsvorganges oder in Pausen. Die Exhalation erfolgt über ein Filter 53 und dann entweder über eine Absaugung oder ein Exspirationsventil 64. Das Exspirationsfilter bietet den Vorteil, dass der Patient mit stärkerem Fluss ausatmen kann als die Absaugvorrichtung absaugt. Außerdem kann er bei einem Ausfall der Absaugvorrichtung durch Weiteratmen die kontaminierte Luft aus dem System in den Filter befördern.

## Patentansprüche

1. Bauteil für eine Inhalationsvorrichtung (1) mit:
einer ersten Lufteinlassöffnung (11, 51) und einer ersten Luftauslassöffnung (12, 51), die über einen ersten Strömungskanal (13) für die Inhalation verbunden sind;
einer zweiten Lufteinlassöffnung (14, 52) und einer zweiten Luftauslassöffnung (15, 52), die über einen zweiten Strömungskanal (16) für die Exhalation verbunden sind; und
einem dem zweiten Strömungskanal zugeordneten Filter (17, 53);
wobei die Exhalation über den zweiten Strömungskanal (16) erfolgt.

2. Bauteil nach Anspruch 1, wobei die Exhalation ausschließlich über den zweiten Strömungskanal (16) erfolgt.

3. Bauteil nach Anspruch 1 oder 2, wobei bei Beendigung der Inhalation der erste Strömungskanal (13) verschlossen, und der zweite Strömungskanal (16) freigegeben wird.

4. Bauteil nach Anspruch 1, 2 oder 3, wobei die erste Luftauslassöffnung (12) und die zweite Lufteinlassöffnung (14) zusammenfallen.

5. Bauteil nach einem der Ansprüche 1 bis 4, wobei der erste Strömungskanal (13) und der zweite Strömungskanal (16) ein Drei-Wege-Ventil bilden.

6. Bauteil nach einem der Ansprüche 1 bis 5, wobei die erste Luftauslassöffnung (12) bzw. die zweite Lufteinlassöffnung (14) mit einem Mundstück (20) verbindbar ist.

7. Bauteil nach Anspruch 1 oder 2, wobei die erste Lufteinlassöffnung (51) und die erste Luftauslassöffnung (51) sowie die zweite Lufteinlassöffnung (52) und die zweite Luftauslassöffnung (52) zusammenfallen.

8. Bauteil nach Anspruch 7, wobei das Bauteil eine Inhalationsmaske (50) ist.

9. Bauteil nach einem der Ansprüche 1 bis 6, ferner mit einer Inhalationsmaske (50), mit der Mund und Nase des Benutzers überdeckbar sind.

10. Bauteil nach Anspruch 9, wobei die Inhalationsmaske (50) eine erste Öffnung (51) aufweist, an der der erste Strömungskanal (13) endet.

11. Bauteil nach Anspruch 10, wobei das Mundstück (20) die Inhalationsmaske (50) durchdringt und sich in den durch die Inhalationsmaske (50) und das Gesicht des Benutzers gebildeten Maskeninnenraum (56) erstreckt.

12. Bauteil nach Anspruch 10 oder 11, wobei das Mundstück (20) verschiebbar zwischen einer Inhalationsstellung, in der der Benutzer das Mundstück (20) in den Mund nehmen kann und einer Exhalationsstellung, in der das Mundstück (20) in den Maskeninnenraum (56) zurückgezogen ist, in der ersten Öffnung (51) angeordnet ist.

13. Bauteil nach einem der Ansprüche 9 bis 12, wobei die Inhalationsmaske (50) eine Absaugöffnung (52) aufweist.

14. Bauteil nach Anspruch 7, 8, oder 13, wobei die Öffnung (52) mit einem zweiten Filter (53) und einer damit verbundenen ersten Absaugeinrichtung (54) verbindbar ist.

15. Bauteil nach einem der Ansprüche 9 bis 14, wobei die Inhalationsmaske (50) im Umfangsbereich (55) derart ausgebildet ist, dass im Falle eines Unterdrucks in dem durch die Inhalationsmaske (50) und dem Gesicht des Benutzers gebildeten Maskeninnenraum (56) ein Druckausgleich über den Umfangsbereich (55) ermöglicht wird.

16. Bauteil nach einem der Ansprüche 9 bis 14, wobei die Inhalationsmaske (50) mindestens ein Einwegventil (60) zum Druckausgleich im Maskeninneren (56) aufweist.

17. Bauteil nach einem der Ansprüche 9 bis 16, wobei die Inhalationsmaske (50) ballonförmig ausgebildet ist.

18. Bauteil nach Anspruch 17, wobei das Mundstück (20) die Inhalationsmaske (50) in einem ersten vom Gesicht des Benutzers abgewandten Bereich (57) durchdringt und sich in den Maskeninnenraum (56) erstreckt und ein gegenüberliegender zweiter, dem Gesicht des Benutzers zugewandter Bereich (58) beim Anlegen der Inhalationsmaske (50) von der Mundstückspitze (21) durchdringbar ist.

19. Bauteil nach Anspruch 6, ferner mit einem Schutzelement (22), das das Mundstück (20) in Umfangsrichtung umgibt und sich über die Mundstückspitze (21) hinaus erstreckt.

20. Bauteil nach Anspruch 19, wobei das Schutzelement am der Mundstückspitze gegenüberliegenden Ende dichtend mit dem Mundstück verbunden ist.

21. Bauteil nach Anspruch 19 oder 20, wobei der Überstand des Schutzelementes (22) gegenüber der Mundstückspitze (21) zwischen 5 und 100 mm beträgt.

22. Bauteil nach Anspruch 19, 20 oder 21, wobei das Schutzelement (22) das gesamte Bauteil (19) umgibt.

23. Bauteil nach einem der Ansprüche 19 bis 22, wobei das Schutzelement (22) selbstexpandierend ist.

24. Bauteil nach einem der Ansprüche 19 bis 23, wobei das Schutzelement (22) als Faltenbalg ausgebildet ist.

25. Bauteil nach einem der Ansprüche 1 bis 24, wobei die zweite Luftauslassöffnung (15) mit einer zweiten Absaugvorrichtung (40) verbindbar ist.

26. Bauteil nach einem der Ansprüche 1 bis 25, wobei die erste Lufteinlassöffnung (11) mit einem Vernebler (30) verbindbar ist.

27. Bauteil nach einem der Ansprüche 1 bis 26, wobei das Bauteil Bestandteil des Aerosolgenerators der Inhalationsvorrichtung ist.

28. Inhalationsvorrichtung mit einem ersten Strömungskanal für die Inhalation und einem zweiten Strömungskanal für die Exhalation, wobei Mittel vorgesehen sind, die verhindern, dass vor, während und nach der Inhalation durch den Benutzer von der Inhalationsvorrichtung abgegebener Wirkstoff in die Umgebung entweicht.

29. Inhalationsvorrichtung nach Anspruch 28, wobei die Verhinderungsmittel ein Bauteil (10) nach einem der Ansprüche 1 bis 6 und 9 bis 27 aufweisen; ferner mit:
einem mit der ersten Lufteinlassöffnung (11) verbundenen Vernebler (30); und
einem mit der ersten Luftauslassöffnung (12) und der zweiten Lufteinlassöffnung (14) verbundenen Mundstück (20).

30. Inhalationsvorrichtung nach Anspruch 29, ferner mit einer mit der zweiten Luftauslassöffnung (15) verbundenen Absaugeinrichtung (40).

31. Inhalationsvorrichtung nach Anspruch 28, wobei die Verhinderungsmittel eine Inhalationsmaske (50) mit einer ersten Öffnung (51) für die Inhalation und einer Absaugöffnung (52) aufweisen, ferner mit einem mit der ersten Öffnung (51) verbundenen Vernebler (30) und einer mit der Absaugöffnung (52) verbundenen Absaugeinrichtung (54).

32. Inhalationsvorrichtung nach Anspruch 31, ferner mit einem der ersten Öffnung (51) zugeordneten Mundstück (20).

33. Inhalationsvorrichtung nach Anspruch 32, wobei das Mundstück (20) verschiebbar zwischen einer Inhalationsstellung, in der der Benutzer das Mundstück (20) in den Mund nehmen kann und einer Exhalationsstellung, in der das Mundstück (20) in den Maskeninnenraum (56) zurückgezogen ist angeordnet ist.

34. Inhalationsvorrichtung nach einem der Ansprüche 29 bis 33, ferner mit einem Pufferelement (61) abstromseitig von der Absaugöffnung (52).

35. Inhalationsvorrichtung nach einem der Ansprüche 29 bis 34, wobei die Inhalationsmaske (50) ferner einen Drucksensor (62) aufweist, der mit der Absaugeinrichtung (54) und/oder mit der Inhalationsvorrichtung betrieblich verbunden ist.

36. Steuerverfahren für ein Bauteil nach einem der Ansprüche 1 bis 27, wobei bei Erkennung einer Beendigung des Inhalationsvorgangs der zweite Strömungskanal (16) für die Exhalation freigegeben wird.
